# EUROPEAN PATENT SPECIFICATION

(11) **EP 1 413 283 B1**
(45) Date of publication and mention of the grant of the patent: **20.09.2017**
(21) Application number: 04000245.3
(22) Date of filing: 09.08.2001
(51) Int. Cl.: A61K 6/083

(54) **Dental compositions comprising bisacrylamides and use thereof**
Bisacylamid enthaltende Dentalmassen und deren Verwendung
Compositions dentaires contenant des bisacrylamides et utilisation correspondante

(30) Priority: 11.08.2000 US 224670 P; 07.08.2001 US 310572 P
(43) Date of publication of application: 28.04.2004
(62) Divisional of application: 01962033.5
(73) Proprietor: DENTSPLY International Inc., York, PA 17405-0872 (US)
(72) Inventor: Walz, Uwe, 78465 Konstanz (DE); Klee, Joachim, E., 78315 Radolfzell (DE)
(74) Representative: Hartz, Nikolai

(56) References cited:
- WO-A-92/21314
- WO-A-93/12759
- WO-A1-99/03444
- DE-A- 2 211 128
- GB-A- 1 446 709
- US-A- 4 813 876
- US-A- 5 545 676
- PATENT ABSTRACTS OF JAPAN vol. 010, no. 262 (C-371), 6 September 1986 (1986-09-06) & JP 61 087608 A (GIICHI TADA;OTHERS: 01), 6 May 1986 (1986-05-06)
- FERRUTI P ET AL: "RECENT RESULTS ON FUNCTIONAL POLYMERS AND MACROMONOMERS OF INTERESTAS BIOMATERIALS OR FOR BIOMATERIAL MODIFICATION" BIOMATERIALS, ELSEVIER SCIENCE PUBLISHERS BV., BARKING, GB, vol. 15, no. 15, 1994, pages 1235-1241, XP001053477 ISSN: 0142-9612
- HILL I R C ET AL: "In vitro cytotoxicity of poly(amidoamine)s: relevance to DNA delivery" BBA - GENERAL SUBJECTS, ELSEVIER SCIENCE PUBLISHERS, NL, vol. 1427, no. 2, 19 April 1999 (1999-04-19), pages 161-174, XP004276296 ISSN: 0304-4165

## Description

### Technical background

Since decades the free-radical polymerization used in electrotechnics, electronics, dental industry, is combined with remarkable advantages in these fields. The frequently used acrylates and methacrylates are applied in combination with pigments and fillers or as pure polymerizable resins. It is well-known that during free-radical polymerization some side-reactions take place. One of them is the inhibition of the outer layer of the polymerizable material due to the influence of oxygen. The thickness of this layer depends on the viscosity of the polymerizable material, the degree of filling, the applied temperature and the time of polymerization. Frequently, the oxygen inhibited layer is disadvantageous due to the mechanical properties in this part are insufficient, the abrasion is higher and the toxicological/allergic potential is increased. The polymerization of very small layers is limited due to the oxygen inhibition, for example in case of covering electronic circuits by screen printing or for dental sealing materials or varnishes.

Furthermore, the conventional methacrylates that were used for dental applications are ester compound. Consequently, they hydrolysis under acidic or basic conditions that frequently leads to a long-term failure.

In order to reduce the oxygen inhibited layer different possibilities were suggested. One of them is the today well-known use of carbonyl/amine initiator systems for photochemical polymerization (R.S. Davison, J.W. Goodin, Eur.Polym.J 18 (1982) 597). Dekker used special color initiators that change triplet-oxygen into singulet-oxygen (C. Dekker, Makromol. Chem. 180 (1979) 2027). Furthermore, surface active additives were used (C.R. Morgan, A.D. Ketley, J. Radiat.Curing 7 (1980) 10) or the photochemical SH-En-Addition was applied (C.R. Morgan, F. Magnotta, A.D. Ketley, J.Polym.Sci., Polym. Ed. 15 (1977), 627).

The photochemical polymerization of monoacrylamides was studied by Smets (G. Smets, Bull.Soc.Chim.Belges 71 (1962) 857, G. Oster, J.Amer.Chem.Soc. 79 (1957) 595). A large number of bisacrylamides were described by Ferrutti (P. Ferrutti et al., Polymer 26 (1985) 1336). These bisacrylamides are solids that are soluble in water due to the secondary amide group or they comprises a piperidine group.

A combination of free-radical and Michael addition polymerization was suggest for encapsulation of electronic circuits (DD 295645; invs.: J. Klee, H.-H. Hörhold, I. Scherlitz-Hofmann).

The new synthesized bisacrylamides should be liquids in order to polymerized them without of solvents and furthermore they and the resulting polymers should be insoluble in water.
Dental compositions are also known from WO99/03444.

### Description of the invention

A dental composition that comprises at least one bisacrylamide a polymerizable monomer; at least one amine and/or an initiator; a stabilizer; pigments and an organic and/or inorganic filler and that have an improved hydrolysis stability.

The bisacrylamide are characterized by the following formula: 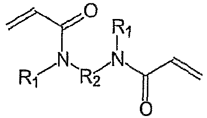 wherein
- R₁: is C₁ to C₁₈ alkyl,
- R₂: is a difunctional substituted or unsubstituted C₁ to C₁₈ alkylene, difunctional substituted or unsubstituted cycloalkylene, difunctional substituted or unsubstituted C₅ to C₁₈ arylene or heteroarylene, difunctional substituted or unsubstituted C₅ to C₁₈ alkylarylene or alkylheteroarylene, difunctional substituted or unsubstituted C₇ to C₃₀ alkylene arylene,

The claimed dental composition preferably contains as polymerizable monomer a mono- or a polyfunctional (meth)-acrylate, such as a polyalkylenoxide di- and poly-(meth)acrylate, an urethane di- and poly(meth) acrylate, a vinyl-, vinylen- or vinyliden-, acrylate- or methacrylate; preferably were used diethyleneglycol dimethacrylate, triethyleneglycol dimethacrylate, 3,(4),8,(9)-dimethacryloyloxymethyltricyclodecane, dioxolan bismethacry-late, glycerol trimethacrylate, furfuryl methacrylate or a monoacrylamide in a content of 5 to 80 wt-%.

Bisacrylamides react with amines in a thermal Michael addition polymerization. Preferably for the addition polymerization are used primary monoamines, disecondary diamines and/or polyamines of the following structure: 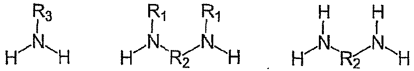 wherein
- R₁: is a substituted or unsubstituted C₁ to C₁₈ alkyl, substituted or unsubstituted cycloalkyl, substituted or unsubstituted C₅ to C₁₈ aryl or heteroaryl, substituted or unsubstituted C₅ to C₁₈ alkylaryl or alkylheteroaryl, substituted or unsubstituted C₇ to C₃₀ alkyl aryl,
- R₂: is a difunctional substituted or unsubstituted C₁ to C₁₈ alkylene, difunctional substituted or unsubstituted cycloalkylene, difunctional substituted or unsubstituted C₅ to C₁₈ arylene or heteroarylene, difunctional substituted or unsubstituted C₅ to C₁₈ alkylarylene or alkylheteroarylene, difunctional substituted or unsubstituted C₇ to C₃₀ alkylene arylene and
- R₃: is a substituted or unsubstituted C₂ to C₁₈ alkylene, substituted or unsubstituted cycloalkyl, substituted or unsubstituted C₅ to C₁₈ aryl or heteroaryl, substituted or unsubstituted C₅ to C₁₈ alkylaryl or alkylheteroaryl, substituted or unsubstituted C₇ to C₃₀ alkyl aryl,

Furthermore the claimed dental composition can contain a polymerization initiator, that preferably is a thermal initiator, a redox-initiator or a photo initiator such as champhor quinone.

In order to avoid a spontaneous polymerization stabilizer are added such as a radical absorbing monomer for example hydrochinonmonomethylether, hydrochinondimethylether, 2,6-di-tert.-butyl-p-cresol.

The dental composition comprises an inorganic filler and/or an organic filler. Preferably inorganic fillers such as La₂O₃, ZrO₂, BiPO₄, CaWO₄, BaWO₄, SrF₂, Bi₂O₃, glasses or an organic fillers, such as polymer granulate or a combination of organic/or inorganic fillers are applied.

The dental composition is preferably usable as dental root canal filling material or as pulp capping material.

### Example 1 (REFERENCE)

N,N'-bisacryloyl-N,N'-dibenzyl-5-oxanonanediamine-1.9: In a 4-necked 1-l-flask equipped with a stirrer, a thermometer and two 50 ml dropping funnels 102.16 g (0.3 mol) of N,N'-dibenzyl-5-oxanonanediamine-1.9 were dissolved in 300 ml of methylenechloride. After cooling to 0-5 °C 57.020 g (0.63 mol) of acryloylchloride dissolved in 30 ml of methylenechloride and 25.20 g (0.63 mol) of NaOH dissolved in 60 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 150 ml of 1 n HCl, 150 ml of 1 n NaHCO₃ and sometimes with 150 ml of deionised water until the water shows a pH-value of approximately 7. Than the organic solution was dried over NaSO₄. Thereafter the NaSO₄ was filtered off and to the solution 0.1346 g of 2,6-di-tert.-butyl-p-cresol were added. The methylenechloride was removed at 40 °C in vacuum and the bisacrylamide was dried.
Yield: 132.6 g (98.5 % of th.), n_{D}²⁰ = 1.5499, η = 2.35 Pa*s, Mₙ (vpo) = 450g/mol

| | | | | |
|---|---|---|---|---|
| C₂₈H₃₆N₂O₃, 448.61 | calc | C 74.97 | H 8.09 | N 6.24 |
| found | | C 74.50 | H 8.09 | N 6.24 |

IR: 1655 cm⁻¹ (CONR), 1620 cm⁻¹ (CH₂=CH-)

| | |
|---|---|
| ¹H-NMR: | 7.4-7.2 (Ph), 6.65/4.52 (CH₂Ph), 5.58/6.38 (CH₂=CH), 3.4-3.2 (CH₂O, CH₂N), 1.6-1.5 (CH₂CH₂) |
| ¹³C-NMR: | 166.69/166.28 (3), 137.60/136.95 (5), 129.66/128.95 (2), 128.80/128.50 (6), 128.35/128.23 (7), 128.16/128.00 (8), 127.27/126.25 (1), 70.40/70.27 (12), 50.99/48.88 (4), 48.07/46.97 (9), 27.43/27.11 (11), 25.43/23.15 (10) |

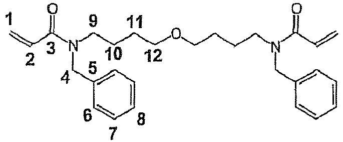

### Addition polymerization:

5.000 g (11.137 mmol) of N,N'-bisacryloyl-N,N'-dibenzyl-5-oxanonanediamine-1.9 and 3.792 g (11.137 mmol) were mixed homogeneously together and reacted for 60 hours at 60 °C. The addition polymer shows the following results obtained by GPC:

| Mₙ g mol⁻¹ | M_{w} g mol⁻¹ | M_{z} g mol⁻¹ | M_{w}/Mₙ | [η] ml g⁻¹ |
|---|---|---|---|---|
| 3615 | 9403 | 16280 | 2.60 | 8.741 |

### Example 2 (REFERENCE)

N,N'-bisacryloyl-N,N'-dibenzylethylenediamine: In a 4-necked 1-l-flask equipped with a stirrer, a thermometer and two 50 ml dropping funnels 29.198 g (0.12 mol) of N,N'-dibenzylethylenediamine were dissolved in 100 ml of methylenechloride. After cooling to 0-5 °C21.991 g (0.24 mol) of acryloylchloride dissolved in 30 ml of methylenechloride and 9.718 g (0.24 mol) of NaOH dissolved in 40 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 100 ml of 1 n HCl, 100 ml of 1 n NaHCO₃ and sometimes with 100 ml of deionised water until the water shows a pH-value of approximately 7. Than the organic solution was dried over NaSO₄. Thereafter the NaSO₄ was filtered off and to the solution 0.028 g of 2,6-di-tert.-butyl-p-cresol were added. The methylenechloride was removed at 40 °C in vacuum and the bisacrylamide was dried.
Yield: 27.9 g (65.9 % of th.), mₚ = 75,5-76,6 °C, Tg = -7.2 °C, Mₙ (vpo) = 350g/mol

| | | | | |
|---|---|---|---|---|
| C₂₂H₂₄N₂O₂, 348.45 | calc. | C 75.83 | H 6.94 | N 8.04 |
| found | C 76.00 | H 7.26 | N 8.05 | |

### Example 3 (REFERENCE)

N,N'-bisacryloyl-N,N'-dibenzyl-4,4'-diaminodicyclohexylamine: In a 4-necked 1-l-flask equipped with a stirrer, a thermometer and two 50 ml dropping funnels 60.551 g (0.16 mol) of N,N'-dibenzyl-4,4'-diaminodicyclohexylamine were dissolved in 150 ml of methylenechloride. After cooling to 0-5 °C 28.061 g (0.31 mol) of acryloylchloride dissolved in 30 ml of methylenechloride and 12.401 g (0.31 mol) of NaOH dissolved in 50 ml of water were added simultaneously under stirring during 1.5 hours so that the temperature remains at 0-5 °C. Thereafter the mixture were stirred at room temperature for additional two hours. Than the reaction mixture were hydrolyzed with 500 ml of ice-water. The organic phase were separated and the aqueous solution were extracted twice with methylenechloride. The collected organic liquids were washed with 100 ml of 1 n HCl, 100 ml of 1 n NaHCO₃ and sometimes with 10 ml of deionised water until the water shows a pH-value of approximately 7. Than the organic solution was dried over NaSO₄. Thereafter the NaSO₄ was filtered off and to the solution 0.077 g of 2,6-di-tert.-butyl-p-cresol were added. The methylenechloride was removed at 40 °C in vacuum and the bisacrylamide was dried.
Yield: 54.0 g (69.9 % of th.), Tg = 47.1 °C

### Application Example 1 (Dental root canal sealer) (REFERENCE)

### Bisacrylamide-Paste

5.0000 g of N,N'-bisacryloyl-N,N'-dibenzyl-5-oxanonanediamine-1.9 of example 1, 3.1642 g of Calciumtungstate, 0.7911 g of Zirconiumoxide, 0.0300 g of Aerosil and 0.0100 g of Fe₂O₃ were mixed homogeneously.

### Amine-Paste

1.8962 g of N,N'-dibenzyl-5-oxanonanediamine-1.9, 0.8423 g of 1-Aminoadamantane, 10.9540 g of Calciumtungstate, 2.7385 g of Zirconiumoxide and 0.3353 g of Aerosil were mixed homogeneously.

Immediately before use both pastes were mixed homogeneously in a ratio of 1/1 (v/v) or 1/1.86 (w/w). The material shows an radio-opacity of 11.5mm/ mm Al.

## Claims

1. A dental composition comprising
(a) at least a bisacrylamide;
(b) a polymerizable monomer,
(c) at least one amine and/or an initiator,
(d) a stabilizer,
(e) pigments, and/or
(f) inorganic filler,
wherein the bisacrylamide is **characterized by** the following formula: 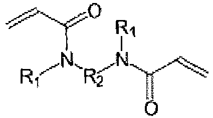 wherein
R₁ is C₁ to C₁₈ alkyl,
R₂ is a difunctional substituted or unsubstituted C₁ to C₁₈ alkylene, difunctional substituted or unsubstituted cycloalkylene, difunctional substituted or unsubstituted C₅ to C₁₈ arylene or heteroarylene, difunctional substituted or unsubstituted C₅ to C₁₈ alkylarylene or alkylheteroarylene, difunctional substituted or unsubstituted C₇ to C₃₀ alkylene arylene.

## Patentansprüche

1. Eine Dentalzusammensetzung, umfassend
(a) mindestens ein Bisacrylamid;
(b) ein polymerisierbares Monomer,
(c) mindestens ein Amin und/oder einen Initiator,
(d) einen Stabilisator,
(e) Pigmente, und/oder
(f) anorganische Füllstoffe,
worin das Bisacrylamid durch folgende Formel charakterisiert ist: 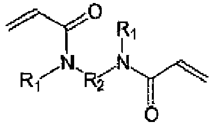 worin
R₁ eine C₁ bis C₁₈ Alkylgruppe ist,
R₂ eine difunktionale substituierte oder unsubstituierte C₁ bis C₁₈ Alkylenegruppe, eine difunktionale substituierte oder unsubstituierte Cycloalkylengruppe, eine difunktionale substituierte oder unsubstituierte C₅ bis C₁₈ Arylengruppe oder Heteroarylengruppe, eine difunktionale substituierte oder unsubstituierte C₅ bis C₁₈ Alkylarylengruppe oder Alkylheteroarylengruppe, oder eine difunktionale substituierte oder unsubstituierte C₇ bis C₃₀ Alkylenarylengruppe ist.

## Revendications

1. Composition dentaire comprenant :
(a) au moins un bisacrylamide ;
(b) un monomère polymérisable,
(c) au moins une amine et/ou un initiateur,
(d) un stabilisant,
(e) des pigments, et/ou
(f) une charge inorganique,
dans laquelle le bisacrylamide est **caractérisé par** la formule suivante : 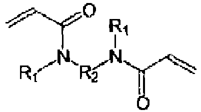 dans laquelle :
R₁ est un groupe alkyle en C₁ à C₁₈,
R₂ est un groupe alkylène en C₁ à C₁₈ substitué ou non substitué difonctionnel, cycloalkylène substitué ou non substitué difonctionnel, arylène ou hétéroarylène en C₅ à C₁₈ substitué ou non substitué difonctionnel, alkylarylène ou alkylhétéroarylène en C₅ à C₁₈ substitué ou non substitué difonctionnel, alkylène-arylène en C₇ à C₃₀ substitué ou non substitué difonctionnel.
